# EUROPÄISCHE PATENTANMELDUNG

(11) **EP 1 314 479 A2**
(43) Veröffentlichungstag der Anmeldung: **28.05.2003**
(21) Anmeldenummer: 02026188.9
(22) Anmeldetag: 25.11.2002
(51) Int. Cl.: B01L 3/00, C12M 1/18

(54) **Vorrichtung für den Transfer flüssiger Proben**

(30) Priorität: 24.11.2001 DE 10157365
(71) Anmelder: GeSIM Gesellschaft für Silizium-Mikrosysteme mbH, 01454 Grosserkmannsdorf (DE)
(72) Erfinder: Howitz, Steffen, Dr., 01159 Dresden (DE); Wegener, Thomas, 16816 Neuruppin (DE); Bürger, Mario, 01796 Pirna (DE)
(74) Vertreter: Patentanwälte Lippert, Stachow, Schmidt & Partner

(57) **Zusammenfassung**

Die Erfindung betrifft eine Vorrichtung für den Transfer flüssiger Proben aus Standardgefäßen in einen mit einer Trägerflüssigkeit gefüllten miniaturisierten Strömungskanal im sub-µl Bereich. Durch die Erfindung soll eine Vorrichtung für den Transfer flüssiger Proben geschaffen werden, mit der es möglich ist, flüssige Proben im sub-µl Bereich totvolumenfrei in einen Strömungskanal zu applizieren und einen extrem geringen Reagenzienverbrauch zu realisieren. Erreicht wird dies dadurch, dass ein eingangsseitig verschließbarer miniaturisierter Strömungskanal (3) mit einem räumlich begrenzten siebartig durchbrochenen Wandbereich (4) vorgesehen ist, auf den eine flüssige Probe (13) mittels einer Mikrodosiereinheit (11) kontaktfrei applizierbar ist und dass die Probe (13) nachfolgend durch eine am Ende (6) dieses Strömungskanals (3) befindliche Pumpe (8) bei eingangsseitig verschlossenem Strömungskanal (3) unter weitgehender Verdrängung der Trägerflüssigkeit einsaugbar ist.

## Beschreibung

Die Erfindung betrifft eine Vorrichtung für den Transfer flüssiger Proben aus Standardgefäßen in einen mit einer Trägerflüssigkeit gefüllten miniaturisierten Strömungskanal im sub-µl Bereich.

Mit der Vorrichtung sollen sämtliche notwendigen Komponenten zur Automatisierung von Screeningprozessen im Durchflußmodus beschrieben werden. Zum Beispiel zum Screening von Zellkulturen, die sich am Detektionsort befinden und dort von einer Nährlösung umspült werden.

In den genannten Fällen ist es erforderlich, Kleinstmengen von Proben in Form von Tropfen, z.B. Zellkulturen oder einzelne zellen, in ein Trägerfluid, z.B. eine Nährlösung, einzubringen. Üblicherweise erfolgt das mit Hilfe der Mikropipettiertechnik, indem einzelne, oder mehrere Tropfen mit der Probe auf einer Nanotiterplatte verteilt werden, um dann entsprechende Untersuchungen vornehmen zu können.

Zunehmend werden jedoch automatisierte Untersuchungsverfahren eingesetzt, indem die Proben mit einem Trägerfluid beispielsweise durch Mikroduchflusszellen (DE 101 04 957 A1) oder Manifolde (WO 00/55618) geleitet werden, in denen Zellmanipulationen vorgenommen werden können.

Zu diesem Zweck ist die Mikrodurchflusszelle oder auch das Manifold mit einem Strömungskanal mit einem Querschnitt im Mikrometerbereich ausgestattet, an dessen Enden Flüssigkeitszuund -abläufe angeordnet sind. Das Manifold hat dabei die Aufgabe, einzelne Komponenten mechanisch definiert zu halten, sie untereinander fluidisch zu verbinden und bei Bedarf elektrisch zu verkoppeln. Derartige Mikrosysteme entstehen durch die Kombination von Sensoren, Aktoren und elektronischen Bauelementen auf dem Manifold, wobei gleichzeitig Einrichtungen zum Zu- und Ableiten von Fluiden integriert sind.

Als besondere Schwierigkeit hat sich hier allerdings gezeigt, dass es schwierig ist, kleinste Probemengen in den mit einem Trägerfluid gefüllten Strömungskanal zu applizieren. Diese Schwierigkeit resultiert aus der Tatsache, dass es bei laminaren Strömungen - um derartige Strömungen handelt es sich in solchen Strömungskanälen - sehr schwer ist, eine Vermischung der applizierten Probe mit dem Trägerfluid zu erreichen. Das bedeutet aber auch, dass es schwierig ist, die Probenzuführung genau zu dosieren.

Aus der WO 199522696 geht eine Mikro-Fluiddiode hervor, die einen Austritt einer im Strömungskanal stehenden oder strömenden Flüssigkeit sicher verhindert und gleichzeitig den Eintritt einer von außen auf die Mikro-Fluiddiode aufzubringenden zweiten Flüssigkeit gewährleistet. Funktionswesentlich ist hier bei der Verwendung von Mikrokappillaren, dass am Ende jeder der Mikrokapillare eine Gas-Flüssigkeits-Grenzfläche vorhanden ist.

Der Erfindung liegt die Aufgabe zugrunde, eine Vorrichtung für den Transfer flüssiger Proben zu schaffen, mit der es möglich ist, flüssige Proben im sub-µl Bereich totvolumenfrei in einen Strömungskanal zu applizieren und einen extrem geringen Reagenzienverbrauch zu realisieren.

Die der Erfindung zugrundeliegende Aufgabe wird dadurch gelöst, dass ein eingangsseitig verschließbarer miniaturisierter Strömungskanal mit einem räumlich begrenzten siebartig durchbrochenen Wandbereich vorgesehen ist, auf den eine flüssige Probe mittels einer Mikrodosiereinheit kontaktfrei applizierbar ist und dass die Probe nachfolgend durch eine am Ende dieses Strömungskanals befindliche Pumpe bei eingangsseitig verschlossenem Strömungskanal unter weitgehender Verdrängung der Trägerflüssigkeit einsaugbar ist.

Durch diese konstruktive Lösung wird gewährleistet, dass die gesamte auf dem durchbrochenen Wandbereich applizierte Flüssigkeitsmenge vollständig in den Strömungskanal transportiert wird, wobei gleichzeitig sichergestellt ist, dass die im Strömungskanal zu Beginn des Einsaugprozesses notwendigerweise befindliche Trägerflüssigkeit weitgehend verdrängt wird. Damit wird eine Verdünnung der Probe verhindert.

Darüber hinaus wird durch die Oberflächenspannung der Trägerflüssigkeit verhindert, dass nach dem Einsaugen der Probe Luft in das System gelangt.

Vorzugsweise wird für den eingangsseitigen Verschluss des miniaturisierten Strömungskanales ein Schaltventil verwendet, das an dessen Eingang angekoppelt ist. Dieses Schaltventil ist zeitgleich mit dem Moment des Betriebes der Pumpe verschließbar und stellt das exakte Einsaugen der Probenflüssigkeit sicher.

Der miniaturisierte Strömungskanal kann kostengünstig mittels mikrotechnischer Fertigungsverfahren aus den Werkstoffen Glas, Silizium, Keramik oder einem Polymer hergestellt werden.

In einer weiteren Ausgestaltung der Erfindung enthält der miniaturisierte Strömungskanal eine Siebstruktur, die den perforierten Wandabschnitt direkt enthält. Diese Siebstruktur kann durch Anwendung der anisotropen Tiefenätzverfahren, oder durch Laserbohren hergestellt werden.

In weiterer Fortführung der Erfindung ist ein Basissubstrat vorgesehen, in welches mehrere miniaturisierte Strömungskanäle mit je einem perforierten Wandabschnitt eingebracht worden sind, so dass der Transfer von flüssigen Proben gleichzeitig in mehrere perforierte Wandabschnitte parallel erfolgen kann.

Schließlich ist eine weitere Ausgestaltung der Erfindung dadurch gekennzeichnet, dass im mit mehreren miniaturisierten Strömungskanälen versehenen Basissubstrat auch je Strömungskanal in einem Abstand zum perforierten Wandabschnitt ein Detektionsort zur parallelen Auswertung der Wechselwirkung zwischen flüssiger Probe und dem jeweiligen Detektionsort vorgesehen ist.

Um den oder die miniaturisierten Strömungskanäle auf einfache Weise und schnell befüllen, entleeren oder reinigen zu können, ist weiterhin ein in x-y-z-Richtung bewegliches Dichtstück vorgesehen, welches einen fluidisch dichten Verschluss des perforierten Wandbereiches ermöglicht.

Zur effektiven Applikation von Proben ist schließlich eine Mikrodosiereinheit vorgesehen, die in x-y-z-Richtung über den perforierten Wandabschnitten der miniaturisierten Strömungskanäle bewegbar angeordnet ist. Diese Mikrodosiereinheit kann aus einer Vielzahl von separat angesteuerten Mikropumpen bestehen, die je Mikropumpe eine kontaktfreie Applikation flüssiger Proben auf je einen separaten, perforierten Wandbereich im entsprechenden miniaturisierten Strömungskanal ermöglicht. Die Anzahl der separat ansteuerbaren Mikropumpen kann bis zu 96 betragen.

Durch die erfindungsgemäße Vorrichtung, die einen totvolumenfreien Transfer flüssiger Proben aus Standardgefäßsystemen in miniaturisierte Strömungskanäle im sub-µl Bereich ermöglicht, kann diese Aufgabe mit dem hohen Anspruch extrem geringen Reagenzienverbrauches pro Analyse verbunden werden. Mit der Erfindung wird eine Verbindung zwischen Mikropipettiertechnik und Mikrodurchflußtechnik auf der Basis einer x-y-z-Robotik geschaffen, die durch die parallelisierte Anordnung den Weg zu neuartigen Hochdurchsatz-Screeningsystemen eröffnet.

Die zugehörigen Zeichnungen zeigen:
- Fig. 1:: eine schematischen Darstellung einer Draufsicht auf eine erfindungsgemäße Vorrichtung;
- Fig. 2:: eine Draufsicht auf eine Parallelanordnung;
- Fig. 3:: einen Querschnitt durch eine erfindungsgemäße Vorrichtung;

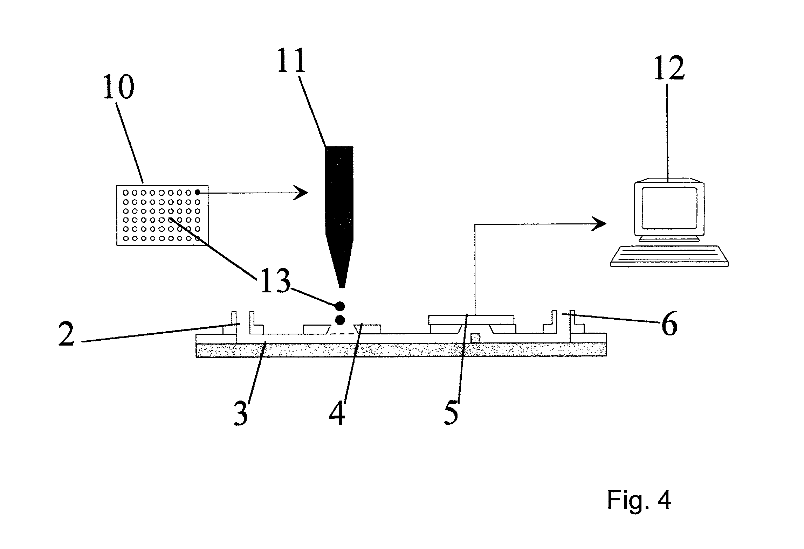

- Fig. 4:: eine schematische Darstellung eine screening Anordnung mit der erfindungsgemäßen Vorrichtung; und
- Fig. 5:: die Anordnung nach Fig. 5 während eines Spülprozesses mit aufgesetztem Dichtstück.

Die Vorrichtung für den totvolumenfreien Transfer flüssiger Proben 13 besteht aus Standardgefäßsystemen 10 in einen mit einer Trägerflüssigkeit gefüllten miniaturisierten Strömungskanal 3 im sub-µl Bereich. Dieser miniaturisierte Strömungskanal 3 ist mit einem räumlich begrenzten siebartig durchbrochenen Wandbereich 4 versehen, auf den die flüssige Probe 13 mittels einer Mikrodosiereinheit 11 kontaktfrei applizierbar ist. Die Probe 13 wird nachfolgend durch eine am Auslass 6 dieses Strömungskanals 3 befindliche Pumpe 8 bei eingangsseitig verschlossenem Strömungskanal 3 unter weitgehender Verdrängung der Trägerflüssigkeit eingesaugt. Die Pumpe 8 ist über eine Leitung 7 mit dem Auslass 6 verbunden.

Der eingangsseitige Verschluss des Strömungskanales 3 erfolgt durch ein Schaltventil 1, das am Eingang 2 des Strömungsjanales 3 angekoppelt ist. Dieses Schaltventil 1 ist zeitgleich mit dem Moment des Betriebes der Pumpe 8 verschließbar und stellt das exakte Einsaugen der Probenflüssigkeit 13 in den Strömungskanal 3 sicher.

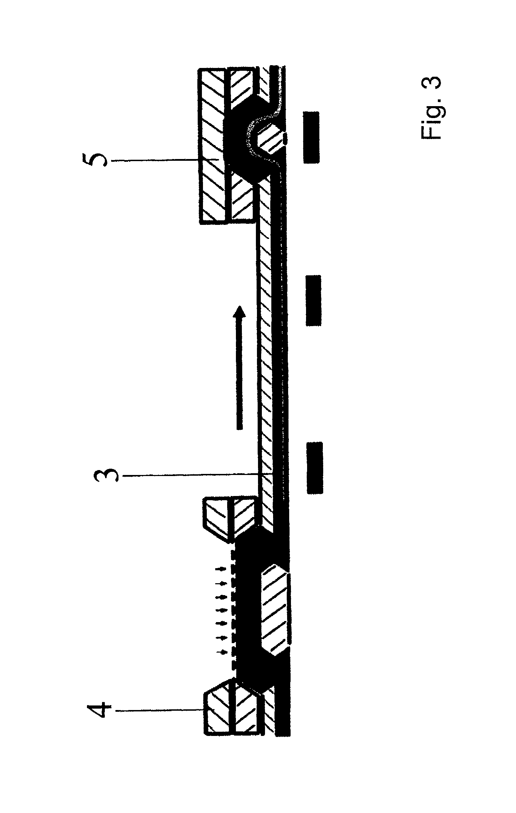

Der miniaturisierte Strömungskanal 3 kann mittels mikrotechnischer Fertigungsverfahren aus den Werkstoffen Glas, Silizium, Keramik oder Polymer hergestellt werden, wobei dieser eine Siebstruktur zur Schaffung des perforierten Wandabschnittes 4 direkt enthält, die durch Anwendung der anisotropen Tiefenätzverfahren oder durch Laserbohren hergestellt worden ist (Fig. 3).

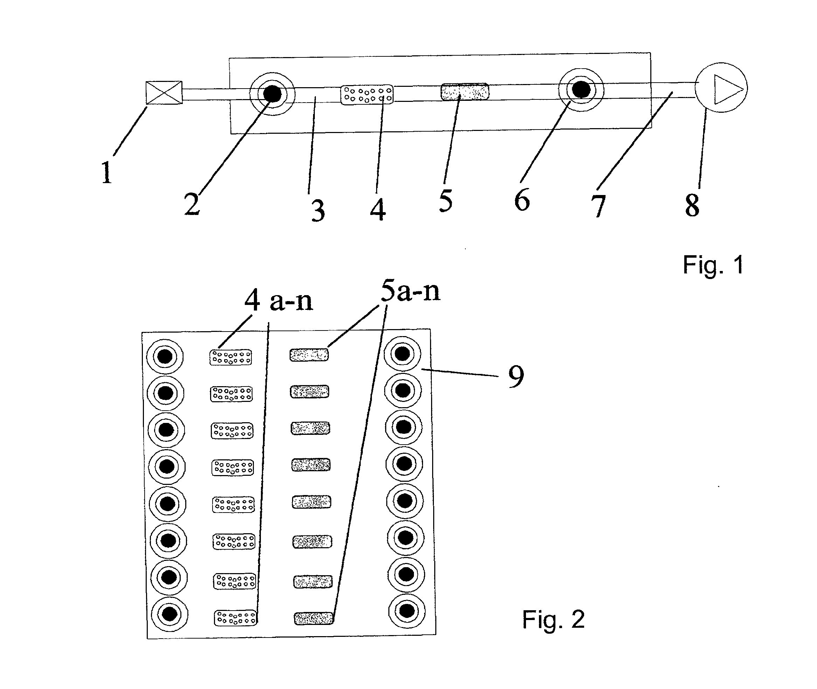

Weiterhin ist ein Basissubstrat 9 vorgesehen, in welches mehrere miniaturisierte Strömungskanäle 3 mit je einem perforierten Wandabschnitt 4a-n eingebracht worden sind (Fig. 1, 2). Dadurch kann ein paralleler Transfer von flüssigen Proben 13 zu einem Zeitpunkt (gleichzeitig) in mehrere perforierte Wandabschnitte 4a - n erfolgen.

Das im mit mehreren miniaturisierten Strömungskanälen 3 versehene Basissubstrat 9 ist weiterhin auch je Strömungskanal in einem Abstand zum perforierten Wandabschnitt 4a-n miteinem Detektionsort 5a-n zur parallelen Auswertung der Wechselwirkung zwischen flüssiger Probe 13 und dem jeweiligen Detektionsort 5a-n versehen.

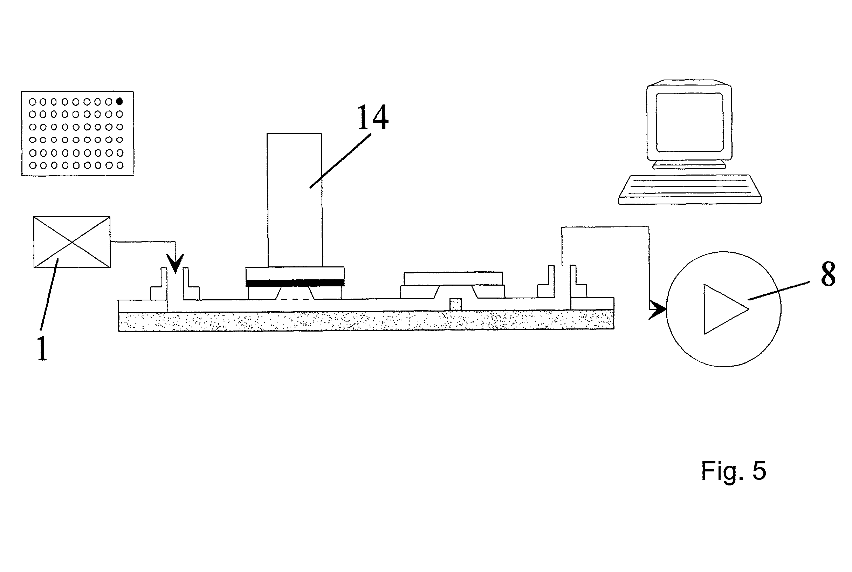

Weiterhin ist ein in x-y-z-Richtung bewegliches Dichtstück 14 vorgesehen, welches einen fluidisch dichten Verschluss des perforierten Wandbereiches 4a-n ermöglicht. Dadurch kann der oder die Mikroströmungskanäle 3 auf einfache Weise befüllt, entleert oder gereinigt werden (Fig. 5).

Fig. 5 zeigt eine Ausführung der Erfindung mit einer Mikrodosiereinheit 11, die in x-y-z-Richtung über den perforierten Wandabschnitten 4a-n der miniaturisierten Strömungskanäle 3 bewegbar angeordnet ist, wodurch eine einfache Probenverteilung auf dem Basissubstrat 9 ermöglicht wird.

Vorteilhafter Weise besteht die Mikrodosiereinheit 11 aus einer Vielzahl von bis zu 96 separat angesteuerten Mikropumpen (Mikrodosiereinheit 11) besteht, die je Mikropumpe eine kontaktfreie Applikation flüssiger Proben 13 auf je einen separaten, perforierten Wandbereich 4a-n im entsprechenden miniaturisierten Strömungskanal 3 ermöglicht.

### Bezugszeichenliste

- 1: Schaltventil
- 2: Eingang
- 3: Strömungskanal
- 4: Wandbereich
- 5: Detektionsort
- 6: Auslass
- 7: Leitung
- 8: Pumpe
- 9: Basissubstrat
- 10: Standardgefäßsystem
- 11: Mikrodosiereinheit
- 12: Auswerte- und Steuereinheit
- 13: Probenflüssigkeit (Mikrotropfen)
- 14: Dichtstück

## Patentansprüche

1. Vorrichtung für den totvolumenfreien Transfer flüssiger Proben (13) aus Standardgefäßsystemen (10) in einen mit einer Trägerflüssigkeit gefüllten miniaturisierten Strömungskanal (3) im sub-µl Bereich, **dadurch gekennzeichnet, dass** ein eingangsseitig verschließbarer miniaturisierter Strömungskanal (3) mit einem räumlich begrenzten siebartig durchbrochenen Wandbereich (4) vorgesehen ist, auf den eine flüssige Probe (13) mittels einer Mikrodosiereinheit (11) kontaktfrei applizierbar ist und dass die Probe (13) nachfolgend durch eine am Ende dieses Strömungskanals befindliche Pumpe (8) bei eingangsseitig verschlossenem Strömungskanal (3) unter weitgehender Verdrängung der Trägerflüssigkeit einsaugbar ist.

2. Vorrichtung nach Anspruch 1, **dadurch gekennzeichnet, dass** für den eingangsseitigen Verschluss des Strömungskanales (3) ein Schaltventil (1) an dessen Eingang (2) angekoppelt ist, dass zeitgleich mit dem Moment des Betriebes der Pumpe (8) verschließbar ist und das exakte Einsaugen der Probenflüssigkeit (13) sicherstellt.

3. Vorrichtung nach Anspruch 1 und 2, **dadurch gekennzeichnet, dass** der miniaturisierte Strömungskanal (3) mittels mikrotechnischer Fertigungsverfahren aus den Werkstoffen Glas, Silizium, Keramik oder Polymer hergestellt worden ist.

4. Vorrichtung nach Anspruch 3, **dadurch gekennzeichnet, dass** der miniaturisierte Strömungskanal (3) eine Siebstruktur zur Schaffung des perforierten Wandabschnittes (4) direkt enthält und diese durch Anwendung der anisotropen Tiefenätzverfahren oder durch Laserbohren hergestellt ist.

5. Vorrichtung nach Anspruch 3 und 4 , **dadurch gekennzeichnet, dass** ein Basissubstrat (9) vorgesehen ist, in welches mehrere miniaturisierte Strömungskanäle (3) mit je einem perforierten Wandabschnitt (4a-n) eingebracht werden und so der Transfer von flüssigen Proben (13) zu einem Zeitpunkt in mehrere perforierte Wandabschnitte (4a-n) parallel erfolgen kann.

6. Vorrichtung nach Anspruch 3, 4 und 6 , **dadurch gekennzeichnet, dass** im mit mehreren miniaturisierten Strömungskanälen (3) versehenen Basissubstrat (9) auch je Strömungskanal in einem Abstand zum perforierten Wandabschnitt (4a-n) ein Detektionsort (5a-n) zur parallelen Auswertung der Wechselwirkung zwischen flüssiger Probe (13) und dem jeweiligen Detektionsort (5a-n) vorgesehen ist.

7. Vorrichtung nach Anspruch 1, **dadurch gekennzeichnet, dass** es ein in x-y-z-Richtung bewegliches Dichtstück (14) gibt, welches einen fluidisch dichten Verschluss des perforierten Wandbereiches (4a-n) ermöglicht. (, wenn der oder die Mikroströmungskanäle befüllt, entleert oder gereinigt werden müssen.)

8. Vorrichtung nach einem der Ansprüche 1 bis 7, **dadurch gekennzeichnet, dass** eine Mikrodosiereinheit (11) vorgesehen ist, die in x-y-z-Richtung über den perforierten Wandabschnitten (4a-n) der miniaturisierten Strömungskanäle (3) bewegbar angeordnet ist.

9. Vorrichtung nach Anspruch 1, **dadurch gekennzeichnet, dass** die Mikrodosiereinheit (11) aus einer Vielzahl von (bis zu 96) separat angesteuerten Mikropumpen besteht, die je Mikropumpe eine kontaktfreie Applikation flüssiger Proben (13) auf je einen separaten, perforierten Wandbereich (4a-n) im entsprechenden miniaturisierten Strömungskanal (3) ermöglicht.
